# EUROPEAN PATENT SPECIFICATION

(11) **EP 3 148 600 B1**
(45) Date of publication and mention of the grant of the patent: **07.08.2019**
(21) Application number: 15726137.1
(22) Date of filing: 29.05.2015
(51) Int. Cl.: A61L 27/26, A61L 27/52, A61L 27/54, A61K 8/67, A61K 8/73, A61K 9/00, A61K 9/06, A61K 47/36, A61K 47/40, A61K 47/50, A61K 31/07, C08B 37/16, C08B 37/02, C08B 37/08

(54) **CYCLODEXTRIN-GRAFTED HYALURONIC ACID CROSSLINKED WITH DEXTRAN AND USES THEREOF**
MIT DEXTRAN VERNETZTE CYCLODEXTRIN-GEPFROPFTE HYALURONSÄURE UND VERWENDUNGEN DAVON
ACIDE HYALURONIQUE À GREFFE DE CYCLODEXTRINE RÉTICULÉ AVEC UN DEXTRANE ET SES UTILISATIONS

(30) Priority: 29.05.2014 US 201462004293 P; 18.12.2014 EP 14198951
(43) Date of publication of application: 05.04.2017
(73) Proprietor: Galderma S.A., 6330 Cham (CH)
(72) Inventor: BOITEAU, Jean-Guy, 06650 Opio (FR); LÄRKNER, Helena, 752 65 Uppsala (SE)
(74) Representative: AWA Sweden AB
(86) International application number: PCT/EP2015/061999
(87) International publication number: WO 2015/181365

(56) References cited:
- WO-A1-00/46252
- US-A1- 2002 192 182
- US-A1- 2013 231 474
- US-A1- 2014 094 433
- LAURA PESCOSOLIDO ET AL: "Hyaluronic Acid and Dextran-Based Semi-IPN Hydrogels as Biomaterials for Bioprinting", BIOMACROMOLECULES, vol. 12, no. 5, 9 May 2011 (2011-05-09), pages 1831-1838, XP055207927, ISSN: 1525-7797, DOI: 10.1021/bm200178w
- JIN R ET AL: "Enzymatically-crosslinked injectable hydrogels based on biomimetic dextran@?hyaluronic acid conjugates for cartilage tissue engineering", BIOMATERIALS, ELSEVIER SCIENCE PUBLISHERS BV., BARKING, GB, vol. 31, no. 11, 1 April 2010 (2010-04-01) , pages 3103-3113, XP026933236, ISSN: 0142-9612, DOI: 10.1016/J.BIOMATERIALS.2010.01.013 [retrieved on 2010-02-08]
- SOLTES L ET AL: "Cyclodextrin derivative of hyaluronan", CARBOHYDRATE POLYMERS, APPLIED SCIENCE PUBLISHERS, LTD. BARKING, GB, vol. 39, no. 1, 1 May 1999 (1999-05-01), pages 17-24, XP004160582, ISSN: 0144-8617, DOI: 10.1016/S0144-8617(98)00135-0
- ZAWKO S A ET AL: "Drug-binding hydrogels of hyaluronic acid functionalized with beta-cyclodextrin", JOURNAL OF BIOMEDICAL MATERIALS RESEARCH. PART A, JOHN WILEY & SONS, INC, US, vol. 87, no. 4, 15 December 2008 (2008-12-15), pages 1044-1052, XP002728107, ISSN: 1552-4965, DOI: 10.1002/JBM.A.31845 [retrieved on 2008-02-06]
- CHARLOT A ET AL: "Controlled synthesis and inclusion ability of a hyaluronic acid derivative bearing beta-cyclodextrin molecules", BIOMACROMOLECULES, AMERICAN CHEMICAL SOCIETY, US, vol. 7, no. 3, 1 March 2006 (2006-03-01), pages 907-913, XP002728110, ISSN: 1525-7797, DOI: 10.1021/BM0507094 [retrieved on 2006-02-10]

## Description

### Technical field of the invention

The present invention relates to the field of hydrogels containing cross-linked polysaccharides and the use of such hydrogels in medical and/or cosmetic applications. More specifically, the present invention deals with cross-linked hydrogels containing hyaluronic acid and dextran, functionalized with cyclodextrin.

### Background of the invention

One of the most widely used biocompatible polymers for medical use is hyaluronic acid (HA). It is a naturally occurring polysaccharide belonging to the group of glycosaminoglycans (GAGs). Hyaluronic acid and the other GAGs are negatively charged heteropolysaccharide chains which have a capacity to absorb large amounts of water. Hyaluronic acid and products derived from hyaluronic acid are widely used in the biomedical and cosmetic fields, for instance during viscosurgery and as a dermal filler.

Water-absorbing gels, or hydrogels, are widely used in the biomedical field. They are generally prepared by chemical crosslinking of polymers to infinite networks. While native hyaluronic acid and certain crosslinked hyaluronic acid products absorb water until they are completely dissolved, crosslinked hyaluronic acid gels typically absorb a certain amount of water until they are saturated, i.e. they have a finite liquid retention capacity, or swelling degree.

Since hyaluronic acid is present with identical chemical structure except for its molecular mass in most living organisms, it gives a minimum of reactions and allows for advanced medical uses. Crosslinking and/or other modifications of the hyaluronic acid molecule is necessary to improve its duration *in vivo.* Furthermore, such modifications affect the liquid retention capacity of the hyaluronic acid molecule. As a consequence thereof, hyaluronic acid has been the subject of many modification attempts.

Another widely used biocompatible polymer is dextran. Dextran is a complex, branched glucan composed of chains of varying lengths (from 3 to 2000 kD). The straight chain consists of α-1,6 glycosidic linkages between glucose molecules, while branches begin from α-1,3 linkages.

Cyclodextrins (sometimes called cycloamyloses), also referred to herein as CDs, are a family of compounds made up of sugar molecules bound together in a ring (cyclic oligosaccharides). Cyclodextrins are produced from starch by means of enzymatic conversion. Typically, cyclodextrins are constituted by 6-8 glucopyranoside units, and have a structural conformation resembling toroids with the primary hydroxyl groups of the glucopyranoside units arranged along the smaller opening of the toroid and the secondary hydroxyl groups of the glucopyranoside units arranged along the larger opening of the toroid. Because of this arrangement, the interior of the toroids is considerably less hydrophilic than the aqueous environment and thus able to host other hydrophobic molecules. In contrast, the exterior is sufficiently hydrophilic to impart cyclodextrins (or their complexes) water solubility.

When a hydrophobic molecule (the guest) is contained, fully or partially, within the interior of the cyclodextrin (the host), this is referred to as an inclusion complex or guest/host complex. The formation of the guest/host complex can greatly modify the physical and chemical properties of the guest molecule, mostly in terms of water solubility. This is a reason why cyclodextrins have attracted much interest in pharmaceutical applications: because inclusion compounds of cyclodextrins with hydrophobic molecules are able to penetrate body tissues, these can be used to release biologically active compounds under specific conditions. In most cases the mechanism of controlled degradation of such complexes is based on change of pH, leading to the cleavage of hydrogen or ionic bonds between the host and the guest molecules. Other mechanisms for the disruption of the complexes include heating or action of enzymes able to cleave α-1,4 linkages between glucose monomers.
Pescosolido, L. et al. discloses Hyaluronic acid dextran-based semi-IPN hydrogels as biomaterials for bioprinting (Biomacromolecules (2012, 12, 5, 1831-1838). Jin R et al. discloses enzymatically injectible hydrogels based on biomimetic dextran-hyaluronic acid conjugates for cartilage tissue engineering (Biomaterials (2010), 31, 11, 3103-3113. US 2002/0192182 A1 discloses a hydrogel in which varying ratios of acryloyl-modified dextran molecules and acryloyl-modified hyaluronan products are crosslinked to form a hydrogel conjugate. US 2014/094433 discloses hydrogel compositions that include a hysluronic acid, a vitamin A, and a cyclodextrin and may be used as medical or cosmetic fillers, implants, or creams. Soltes L. et al discloses a cyclodextrin derivative of hyaluronan (Carbohydrate polymers (1999), 39, 17-24). Zawko S A et al discloses drug-binding hydrogels of hyaluronic acid functionalized with beta-cyclodextrin (J. of biomedical materials research (2008), 87, 4, 1044-1052. Chariot A et al discloses controlled synthesis and inclusion ability of a hyaluronic acid derivative bearing beta-cyclodextrin molecules (Biomacromolecules, American Chemical Society (2006), 7, 907-913.) US 2013/231474 A1 discloses polysaccharides grafted with a unit including a carbon-carbon double bond, to polysaccharides grafted with a unit including a carbon-carbon double bond functionalized by a thioether unit. WO 00/46252 discloses a process for the production of hyaluronic acid (HA) derivatives cross-linked with another polymer, in particular multiple e.g. double cross-linked hyaluronic acid derivatives.

### Summary of the invention

It is an object of the present invention to provide improved formulations for administration of pharmaceutical and/or cosmetic substances.

In particular, it is an object of the present invention to provide a cross-linked hydrogel product having a significant amount of grafted cyclodextrins.

It is also an object of the present invention to provide a process for preparing improved formulations for administration of pharmaceutical and/or cosmetic substances.

In particular, it is an object of the present invention to provide a process for providing a cross-linked hydrogel product having a significant amount of grafted cyclodextrins.

For these and other objects that will be evident from this disclosure, the present invention provides according to a first aspect a hydrogel product comprising (a) one or more cyclodextrin molecules grafted to hyaluronic acid and (b) dextran, wherein the cyclodextrin-grafted hyaluronic acid is cross-linked to the dextran.

It is advantageous to graft the cyclodextrin molecules on the hyaluronic acid prior to the cross-linking between the polymers, since this makes the final gel product more homogenous with respect to cyclodextrin distribution. In certain applications, for instance slow release administration of drug molecules, it may be particularly useful to have an even distribution of the cyclodextrins throughout the gel product.

In certain preferred embodiments, the dextran is cross-linked to the cyclodextrin-grafted hyaluronic acid by ether bonds.

In some preferred embodiments, the one or more cyclodextrin molecules are grafted onto the hyaluronic acid by amide bonds.

According to a preferred embodiment, the cross-linked hydrogel product is in the form of gel particles having an average size in the range of 0.01-5 mm, preferably 0.1-0.8 mm.

According to one preferred embodiment, the dextran and the hyaluronic acid chains are cross-linked to each other via a linking group which is derived from a bi- or polyfunctional cross-linking agent, such as a diglycidyl ether, e.g. 1,4-butanediol diglycidyl ether (BDDE).

According to a preferred embodiment, the cyclodextrin molecule contains a linking group having an amino group, and wherein the linking group of the cyclodextrin molecule forms said amide bond with a carboxyl group of the hyaluronic acid. Certain linking groups contain a C₁₋₆ alkyl or C₁₋₆ alkyl linker.

The linking group can also be an amino group. A preferred group of cyclodextrin molecules are aminocyclodextrins, such as α-, β- or γ-cyclodextrins, of which 2-aminocyclodextrin, 3-aminocyclodextrin and 6-aminocyclodextrin are preferred.

According to one preferred embodiment, the hydrogel product is further comprising a guest molecule capable of forming a guest-host complex with the cyclodextrin molecule acting as a host. The guest molecule may be selected from drugs and/or biologically active substances used in the treatment of disorders in the field of dermatology, aesthetics, ophthalmology, gynaecology, oncology, angiology, neurology, orthopaedics, rheumatology or aesthetic dermatology.

According to another aspect, the present invention provides a process of preparing a hydrogel product comprising the steps of:
(a) providing (i) hyaluronic acid, (ii) dextran, and (iii) one or more cyclodextrin molecules;
(b) grafting the one or more cyclodextrin molecules onto the hyaluronic acid to form a cyclodextrin-grafted hyaluronic acid; and
(c) cross-linking the cyclodextrin-grafted hyaluronic acid to the dextran using a bi- or polyfunctional cross-linking agent.

It has been realized that grafting of the cyclodextrin molecules onto the hyaluronic acid prior to the cross-linking with dextran is associated with a more homogenous cyclodextrin distribution in the final gel product.

In certain preferred embodiments, the cross-linking of step (c) provides ether bonds between the dextran and the cyclodextrin-grafted hyaluronic acid.

In some preferred embodiments, the grafting of step (b) provides amide bonds between the one or more cyclodextrin molecules and the hyaluronic acid.

According to a preferred embodiment, the cyclodextrin molecule contains a linking group having an amino group, and wherein the linking group of the cyclodextrin molecule forms said amide bond with a carboxyl group of the hyaluronic acid. Certain linking groups contain a C₁₋₆ alkyl or C₁₋₆ alkyl linker.

The linking group can also be an amino group. A preferred group of cyclodextrin molecules are aminocyclodextrins, such as α-, β- or γ-cyclodextrins, of which 2-aminocyclodextrin, 3-aminocyclodextrin and 6-aminocyclodextrin are preferred.

In a preferred embodiment, the grafting of step (b) involves:
(i) activating the carboxyl groups on the hyaluronic acid with a peptide coupling reagent to form an activated hyaluronic acid; and
(ii) coupling the linking group of the one or more cyclodextrin molecules to the carboxyl groups of the activated hyaluronic acid by amide bonds.

In one preferred embodiment, the peptide coupling reagent is selected from the group consisting of triazine-based coupling reagents, carbodiimide coupling reagents, imidazolium-derived coupling reagents, Oxyma and COMU. The peptide coupling reagent is preferably a triazine-based coupling reagent, such as 4-(4,6-dimethoxy-1,3,5-triazin-2-yl)-4-methylmorpholinium chloride (DMTMM).

According to a related aspect, the present invention provides a process of preparing a formulation of a guest molecule capable of forming a guest-host complex with a cyclodextrin host molecule, comprising the steps:
(a) providing (i) hyaluronic acid, (ii) dextran and (ii) one or more cyclodextrin host molecules capable of forming a guest-host complex with the guest molecule;
(b) grafting the one or more cyclodextrin host molecules onto the hyaluronic acid to form a cyclodextrin-grafted hyaluronic acid; and
c) cross-linking the cyclodextrin-grafted hyaluronic acid to the dextran using a bi- or polyfunctional cross-linking agent to form a cross-linked hydrogel.
d) bringing a solution of the guest molecule into contact with the cyclodextrin host molecules grafted onto the hyaluronic acid under conditions allowing for the formation of a guest-host complex between the cyclodextrin host molecules and the guest molecule, and optionally
e) recovering the guest-host complex bound to the cross-linked hydrogel.

In certain preferred embodiments, the cross-linking of step (c) provides ether bonds between the dextran and the cyclodextrin-grafted hyaluronic acid.

In some preferred embodiments, the grafting of step (b) provides amide bonds between the one or more cyclodextrin molecules and the hyaluronic acid.

The guest molecule may be selected from drugs and/or biologically active substances used in the treatment of disorders in the field of dermatology, aesthetics, ophthalmology, gynaecology, oncology, angiology, neurology, orthopaedics, rheumatology or aesthetic dermatology.

Other aspects and preferred embodiments of the present invention will be evident from the following detailed disclosure of the invention and the appended claims.

### Brief description of the drawing

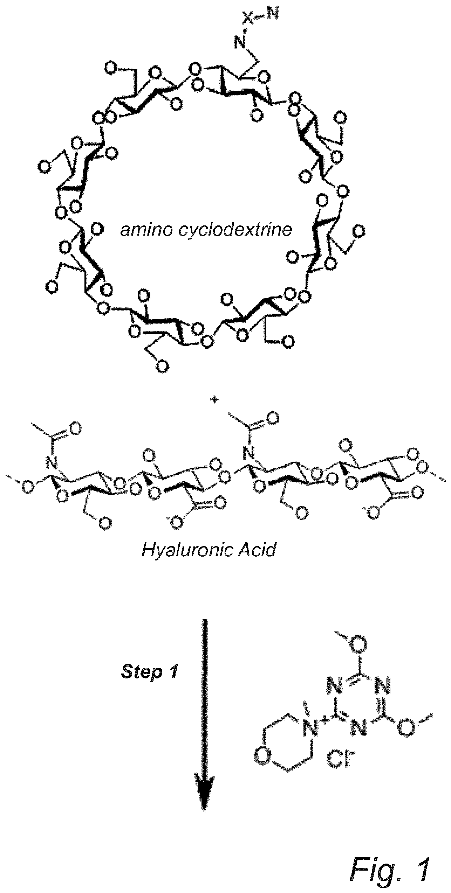

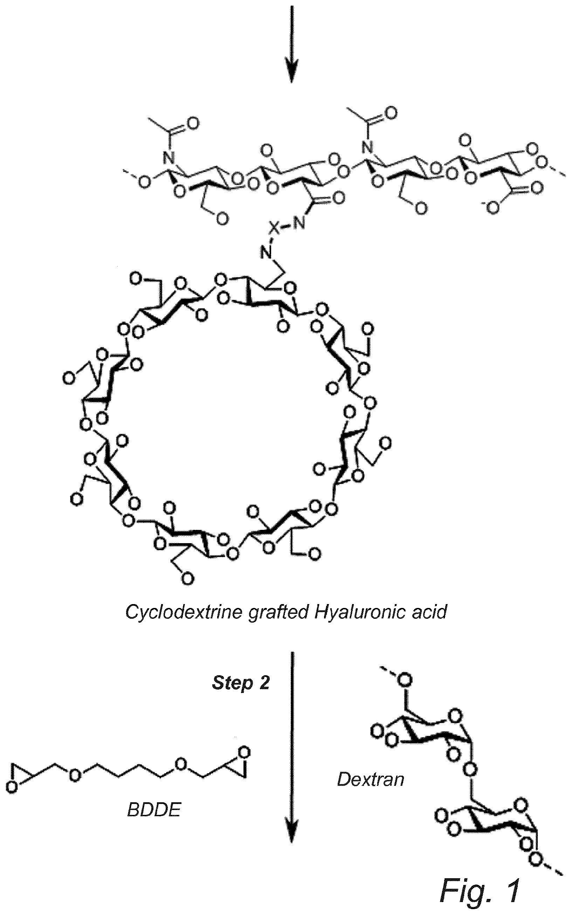

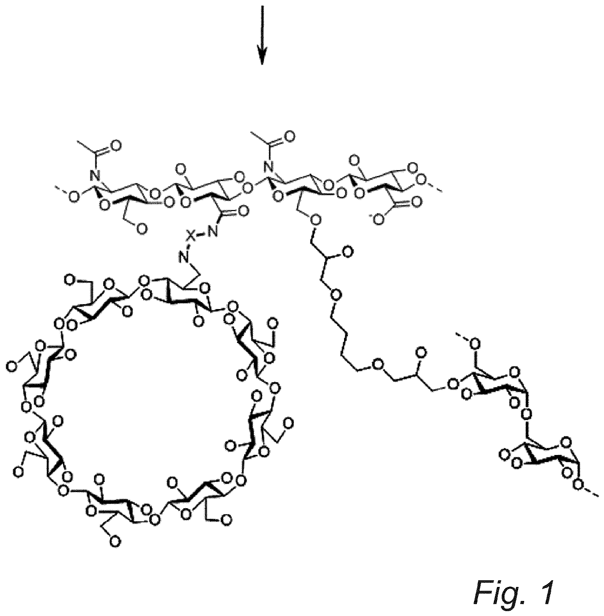

Fig. 1 shows reaction of HA with amino-cyclodextrin and DMTMM (step 1, grafting), followed by reaction with dextran and BDDE (step 2, cross-linking).

### Detailed description of the invention

The present invention generally provides a hydrogel of a cross-linked polymer mixture with grafted cyclodextrin molecules grafted. The hydrogel product is comprising one or more cyclodextrin molecules which are grafted onto the hyaluronic acid, preferably by amide bonds. The hydrogel product is further comprising dextran which is cross-linked to the cyclodextrin-grafted hyaluronic acid, preferably by ether bonds.

In a preferred embodiment, the invention deals with production of hyaluronic acid (HA) grafted with cyclodextrins, e.g. amino-cyclodextrins using peptidic coupling, wherafter this functionalized polysaccharide is linked to dextran by the use of a diepoxide linker like butanediol diglycidyl ether (BDDE).

An amino-cyclodextrin can be linked to HA by an amide bond via a spacer or without a spacer. The optional spacer can be represented by X-N and can be any of the linker known by a person skilled in the art. X can also be equal to zero, that means in this case that the amino-cyclodextrin is directly linked to HA by the amide bond. This amide bond can be realized by reaction of an amino group of amino-cyclodextrin with the carboxylic group of glucuronic acids using a peptidic coupling reagent like DMTMM as depicted in Fig. 1, step 1.

The cyclodextrin grafted HA can be linked to dextran by an ether bond between one hydroxyl group of HA with the hydroxyl group of dextran. This ether bond between the two components can be realized by means of a diepoxide linker like butanediol diglycidyl ether (BDDE) as depicted in Fig. 1, step 2, which also provides a spacer between the HA and the dextran.

Unless otherwise provided, the term "dextran" encompasses all variants and combinations of variants of dextran, of various chain lengths and charge states, as well as with various chemical modifications. Dextran is a complex, branched glucan composed of chains of varying lengths (from 3 to 2000 kD). The straight chain consists of α-1,6 glycosidic linkages between glucose molecules, while branches begin from α-1,3 linkages. Dextran is a bacterial polysaccharide and may be synthesized from sucrose by certain lactic-acid bacteria, for example, *Leuconostoc mesenteroides* and *Streptococcus mutans.*

Unless otherwise provided, the term "hyaluronic acid" encompasses all variants and combinations of variants of hyaluronic acid, hyaluronate or hyaluronan, of various chain lengths and charge states, as well as with various chemical modifications. That is, the term also encompasses the various hyaluronate salts of hyaluronic acid with various counter ions, such as sodium hyaluronate. Various modifications of the hyaluronic acid are also encompassed by the term, such as oxidation, e.g. oxidation of -CH₂OH groups to -CHO and/or -COOH; periodate oxidation of vicinal hydroxyl groups, optionally followed by reduction, e.g. reduction of -CHO to -CH₂OH or coupling with amines to form imines followed by reduction to secondary amines; sulphation; deamidation, optionally followed by deamination or amide formation with new acids; esterification; and deacetylation. Other examples of modifications are isourea, hydrazide, bromocyan, monoepoxide and monosulfone couplings.

The hyaluronic acid can be obtained from various sources of animal and non-animal origin. Sources of non-animal origin include yeast and preferably bacteria. The molecular weight of a single hyaluronic acid molecule is typically in the range of 0.1-10 MDa, but other molecular weights are possible.

A critical feature of the present invention is that the cyclodextrin molecules are grafted onto the hyaluronic acid chains prior to their cross-linking to the dextran chains, preferably by amide bonds to the carboxyl groups of the hyaluronic acid. Grafting of the cyclodextrin molecules onto the hyaluronic acid prior to the cross-linking with dextran is associated with a more homogenous cyclodextrin distribution in the final gel product. The resulting cross-linked hydrogel product is thus suitable for slow-release applications. It is preferred that the cyclodextrin molecules and the disaccharides of the cross-linked hyaluronic acid have a molar ratio of 0.1-50%, preferably 1-20%, and more preferably 8-12%.

The cyclodextrin molecules are useful as carriers (hosts) for a pharmaceutical agent (guest). When a pharmaceutical agent (the guest) is contained, fully or partially, within the interior of the cyclodextrin (the host), this is referred to as an inclusion complex or guest/host complex. The cyclodextrin may then release the pharmaceutical agent under specific conditions, e.g. due to change in pH leading to the cleavage of hydrogen or ionic bonds between the host and the guest molecules.

The cyclodextrin molecules are attached to the the hyaluronic acid component of the cross-linked polymer mixture in order to reduce migration of the cyclodextrin (or guest/host complex) from the site of administration, e.g. injection. In this way, the site of release of the pharmaceutical agent from the cyclodextrin can be controlled.

Also, in order to increase temporal control of the release of the pharmaceutical agent, it has been found that the influence of cleavage of the bonds between the cyclodextrin (or guest/host complex) and the cross-linked polymer mixture should be minimized. In other words, it is desired that the release of the pharmaceutical agent is, as far as possible dependent on the physical release from the cyclodextrin rather than on chemical degradation.

In preferred hydrogel products, the cyclodextrin molecules are attached to the hyaluronic acid by amide bonds. The use of amide bonds in the cyclodextrin-hyaluronic acid linkage (graft) has been found to be advantageous compared to e.g. ester bonds, since the amide bond is more stable to degradation in the subsequent cross-linking step as well as *in vivo.* The use of a less stable bond between the hyaluronic acid and cyclodextrin molecules could lead to premature loss of cyclodextrin (or guest/host complex) from the site of injection.

The cyclodextrin of the hydrogel product may in practice be any cyclodextrin capable of acting as the host molecule in a guest/host complex together with a pharmaceutical agent. Cyclodextrins may generally be constituted by 5-32 glucopyranoside units. However, cyclodextrins constituted by 6-8 glucopyranoside units are generally preferred for the formation of guest/host complexes with pharmaceutical agents. Cyclodextrins constituted by 6, 7 and 8 glucopyranoside units are often referred to as α-, β- and γ-cyclodextrins respectively. According to an embodiment, the cyclodextrin molecules are constituted by 6 glucopyranoside units (α-cyclodextrin). According to an embodiment, the cyclodextrin molecules are constituted by 7 glucopyranoside units (β-cyclodextrin). According to an embodiment, the cyclodextrin molecules are constituted by 8 glucopyranoside units (γ-cyclodextrin).

Cyclodextrins are often chemically modified in order to improve their solubility in water and/or to optimize their performance in a specific application. The term cyclodextrin, α-cyclodextrin, β-cyclodextrin and γ-cyclodextrin, as used herein is also intended to encompass the functionally equivalent variants or derivatives thereof. Examples of such chemically modified cyclodextrins include, but are not limited to, hydroxypropyl and methyl cyclodextrins.

Examples of modified α-cyclodextrins for use with the hydrogel composition include, but are not limited to, hydroxypropyl-α-cyclodextrin.

Examples of modified β-cyclodextrins for use with the hydrogel composition include, but are not limited to, hydroxypropyl-β-cyclodextrin; 2,6-di-O-methyl-β-cyclodextrin; 6-O-maltosyl-β-cyclodextrin; 2-hydroxypropyl-β-cyclodextrin; methyl-β-cyclodextrin; sulfobutyl-β-cyclodextrin; monochlorotriazinyl-β-cyclodextrin; heptakis (2-ω-amino-O-oligo (ethylene oxide)-6-hexylthio)-β-cyclodextrin; ethylenediamino or diethylenetriamino bridged bis(β cyclodextrin)s; randomly methylated β-cyclodextrin; sulfobutyl ether-β-cyclodextrin; and monochlorotriazinyl-β-cyclodextrin.

Examples of modified γ-cyclodextrins for use with the hydrogel composition include, but are not limited to, γ-cyclodextrin C6, and 2,3-di-O-hexanoyl-y cyclodextrin. Further additional modified cyclodextrins are also shown in Tables 1-3 herein.

According to the invention, it is preferred that the cyclodextrin molecule contains a linking group having an amino group. The linking group is covalently attached to the cyclodextrin backbone and may include a spacer molecule, such as C₁₋₆ alkyl or C₁₋₄ alkyl. The linking group of the cyclodextrin molecule preferably forms an amide bond with a carboxyl group of the hyaluronic acid. It is preferred that the linking group has or is an amino group. In a preferred embodiment, the cyclodextrin molecule is an aminocyclodextrin molecule, and the amino group of the aminocyclodextrin molecule forms the amide bond with a carboxyl group of the hyaluronic acid.

A preferred group of cyclodextrin molecules are aminocyclodextrin molecules constituted by 6-8 glucopyranoside units, such as 6 glucopyranoside units (α-cyclodextrin), 7 glucopyranoside units (β-cyclodextrin) or 8 glucopyranoside units (γ-cyclodextrin). It is then furthermore preferred that the cyclodextrin molecule is selected from the group consisting of 2-aminocyclodextrin, 3-aminocyclodextrin and 6-aminocyclodextrin, preferably from the group consisting of 3-aminocyclodextrin and 6-aminocyclodextrin. A preferred cyclodextrin molecule is 6-aminocyclodextrin.

The hydrogel product according to the invention is comprising a cross-linked polymer mixture, consisting of dextran cross-linked to cyclodextrin-grafted (functionalized) hyaluronic acid. It follows that there are covalent cross-links between the dextran chains and the hyaluronic acid chains, the latter of which have previously been grafted with the cyclodextrin(s). It is understood that in the general case, the cross-linking process between dextran and hyaluronic acid is complex and will in addition to these cross-links also provide covalent cross-links between the hyaluronic acid chains as well as between the dextran chains. Together, this creates a continuous network of dextran and hyaluronic acid molecules which is held together by the covalent crosslinks, physical entangling of the polymer chains and various interactions, such as electrostatic interactions, hydrogen bonding and van der Waals forces.

In certain embodiments, the concentration of the cross-linked polymer mixture is in the range of 1 to 100 mg/ml. In some embodiments the concentration of the cross-linked polymer mixture is in the range of 2 to 50 mg/ml. In specific embodiments the concentration of the cross-polymer mixture is in the range of 5 to 30 mg/ml or in the range of 10 to 30 mg/ml.

The weight ratio between the dextran and the hyaluronic acid in the cross-linked polymer mixture may vary to large extent, typically from 100:1 to 1:100, such as from 10:1 to 1:10, e.g. from 5:1 to 1:5.

The crosslinked hydrogel product is preferably biocompatible. This implies that no, or only very mild, immune response occurs in the treated individual. That is, no or only very mild undesirable local or systemic effects occur in the treated individual.

The crosslinked product according to the invention is a gel, or a hydrogel. That is, it can be regarded as a water-insoluble, but substantially dilute crosslinked system of dextran and hyaluronic acid molecules when subjected to a liquid, typically an aqueous liquid.

The gel contains mostly liquid by weight and can e.g. contain 90-99.9% water, but it behaves like a solid due to a three-dimensional crosslinked polymer network within the liquid. Due to its significant liquid content, the gel is structurally flexible and similar to natural tissue, which makes it very useful as a scaffold in tissue engineering and for tissue augmentation.

Crosslinking of the dextran to the cyclodextrin-grafted (functionalized) hyaluronic acid may be achieved by modification with a crosslinking agent. The polymer concentrations and the extent of crosslinking affects the mechanical properties, e.g. the elastic modulus G', and stability properties of the gel. Crosslinked polymer gels are often characterized in terms of "degree of modification". The degree of modification of hyaluronic acid-containg gels generally range between 0.1 and 15 mole%. The degree of HA modification (mole%) describes the amount of crosslinking agent(s) that is bound to HA, i.e. molar amount of bound crosslinking agent(s) relative to the total molar amount of repeating HA disaccharide units. The degree of modification reflects to what degree the HA has been chemically modified by the crosslinking agent. Reaction conditions for crosslinking and suitable analytical techniques for determining the degree of modification are all well known to the person skilled in the art, who easily can adjust these and other relevant factors and thereby provide suitable conditions to obtain a degree of modification in the range of 0.1-2% and verify the resulting product characteristics with respect to the degree of modification. A BDDE (1,4-butandiol diglycidylether) crosslinked dextran/hyaluronic acid gel may for example be prepared analogously to the method described in Examples 1, 2 or 7 of published international patent application WO 9704012.

In a preferred embodiment, the hydrogel product is present in the form of a gel crosslinked by a crosslinking agent, wherein the concentration of said polymers is in the range of 10 to 30 mg/ml, and the degree of HA modification with said crosslinking agent is in the range of 0.1 to 2 mole%.

The hydrogels may also comprise a portion of dextran and/or hyaluronic acid which is not crosslinked, i.e not bound to the three-dimensional crosslinked hyaluronic acid network. However, it is preferred that at least 50 % by weight, preferably at least 60 % by weight, more preferably at least 70 % by weight, and most preferably at least 80 % by weight, of the dextran and hyaluronic acid in a gel composition form part of the crosslinked polymer network.

The hydrogel product is present in the form of particles, strings, discs, etc. In a preferred embodiment, the cross-linked hydrogel is in the form of gel particles. The gel particles preferably have an average size in the range of 0.01-5 mm, preferably 0.1-0.8 mm, such as 0.2-0.5 mm or 0.5-0.8 mm.

The hydrogel product may be present in an aqueous solution, but it may also be present in dried or precipitated form, e.g. in a suitable precipitant liquid. The hydrogel product is preferably injectable.

The dextran and the hyaluronic acid chains are preferably cross-linked to each other via a linking group which is derived from a bi- or polyfunctional cross-linking agent. The bi- or polyfunctional crosslinking agent of the hydrogel product connects the dextran to the hyaluronic acid. The bi- or polyfunctional crosslinking agent further acts as a spacer between the dextran and the hyaluronic acid.

The bi- or polyfunctional crosslinking agent comprises two or more functional groups capable of reacting with functional groups of the dextran and the hyaluronic acid, respectively, resulting in the formation of covalent bonds. The bi- or polyfunctional crosslinking agent may for example selected from the group consisting of divinyl sulfone, multiepoxides and diepoxides.

A preferred type of bi- or polyfunctional cross-linking agent is a bis- or polyepoxide, such as a diglycidyl ether. According to an embodiment, the bi- or polyfunctional crosslinking agent comprises two or more glycidyl ether functional groups. The glycidyl ether functional groups react with primary hydroxyl groups of the dextrans and the hyaluronic acid, respectively, resulting in the formation of ether bonds. It follows that when a diglycidyl ether cross-linking agent reacts with the primary hydroxyl groups of these polymers, two ether bonds are formed with an intermediate spacer remaining from the cross-linking agent,

Preferred bi- or polyfunctional cross-linking agent for cross-linking the dextran and the hyaluronic acid chains include 1,4-butanediol diglycidyl ether (BDDE), 1,2-bis(2,3-epoxypropoxy)ethylene (EGDGE) and ethylene glycol diglycidyl ether (EGDE), 1,2-ethanediol diglycidyl ether (EDDE) and diepoxyoctane. A particularly preferred bi- or polyfunctional cross-linking agent is BDDE.

The hydrogel product disclosed herein may advantageously be used for the transport or administration and slow or controlled release of various pharmaceutical or cosmetic substances.

The hydrogel product may also comprise a guest molecule capable of forming a guest-host complex with the cyclodextrin molecule acting as a host. The guest molecule is generally hydrophobic or lipophilic or has a portion/moiety which is hydrophobic or lipophilic. The size and properties of the guest molecule determines which cyclodextrin is suitable as host. Much effort has been invested in the scientific field to determine suitable cyclodextrin host molecules for various pharmaceutical guest molecules. Some of the guest-host complexes identified are presented in Tables 1-3 herein.

The guest molecule is preferably selected from drugs and/or biologically active substances used in the treatment of disorders in the field of dermatology, aesthetics, ophthalmology, gynaecology, oncology, angiology, neurology, orthopaedics, rheumatology or aesthetic dermatology, such as anti-infective agents, antimicrobials, anti-inflammatory agents, cytostatic, cytotoxic, antiviral, anaesthetic, hemostatic, vasoconstrictor agents or growth factors.

According to a preferred embodiment, the guest molecule is a vitamin A molecule, such as retinol or retinoic acid, or an ester or an ether thereof.

The hydrogel product according to the invention is thus useful as a medicament, e.g. in the treatment of a condition susceptible to treatment by said guest molecule.

The hydrogel product is also useful for the manufacture of a medicament for treatment of a condition susceptible to treatment by said guest molecule.

There is also disclosed that the hydrogel product may be useful in a method of treating a patient suffering from a condition susceptible to treatment by a guest molecule, by administering to the patient a therapeutically effective amount of a hydrogel product according to the invention comprising said guest molecule.

According to another aspect, the hydrogel product is useful in a method of cosmetically treating skin, which comprises administering to the skin a hydrogel product according to the invention.

Since the nature of the product obtainable by the processes according to the invention is complex, the product may also be defined as being the result of these processes.

The present invention furthermore provides an advantageous process of preparing a hydrogel product, comprising the steps of:
(a) providing (i) hyaluronic acid, (ii) dextran, and (iii) one or more cyclodextrin molecules;
(b) grafting the one or more cyclodextrin molecules onto the hyaluronic acid to form a cyclodextrin-grafted hyaluronic acid; and
(c) cross-linking the cyclodextrin-grafted hyaluronic acid to the dextran using a bi- or polyfunctional cross-linking agent.

In certain preferred embodiments, the cross-linking of step (c) provides ether bonds between the dextran and the cyclodextrin-grafted hyaluronic acid.

In some preferred embodiments, the grafting of step (b) provides amide bonds between the one or more cyclodextrin molecules and the hyaluronic acid.

Other preferred embodiments of steps (a) and (c) are evident from the description above relating to the resulting product. In particular, step (c) may further comprise size reduction of the cross-linked polymer mixture into particles, strings, discs, etc. In a preferred embodiment, the cross-linked polymer mixture is brought into the form of gel particles. The gel particles are obtained by reaction of dextran, cyclodextrin-grafted hyaluronic acid and a cross-linking reagent, e.g. BDDE under standard conditions. Particle size reduction of the resulting hydrogel followed by precipitation in ethanol induce the formation of such particles. The gel particles preferably have an average swelled size in the range of 0.01-5 mm, preferably 0.1-0.8 mm, such as 0.2-0.5 mm or 0.5-0.8 mm.

It is preferred that the one or more cyclodextrin molecules of step (a) contain a linking group having an amino group, and wherein the linking group of the cyclodextrin molecule forms said amide bond with a carboxyl group of the hyaluronic acid. The linking group may contain a spacer, such as C₁₋₆ alkyl or C₁₋₄ alkyl. The linking group preferably is an amino group. Thus a preferred type of cyclodextrin molecules are aminocyclodextrin molecules as disclosed hereinabove.

In a preferred process, the grafting of step (b) involves:
(i) activating the carboxyl groups on the hyaluronic acid with a peptide coupling reagent to form an activated hyaluronic acid; and
(ii) coupling the linking group of the one or more cyclodextrin molecules to the carboxyl groups of the activated hyaluronic acid by amide bonds.

It has inventively been realized that the use of peptide coupling reagents on hyaluronic acid prior to the cross-linking step allows for an even distribution (grafting) of cyclodextrins in the final product via amide bonds to the cross-linked hyaluronic acid in the final product.

The one or more cyclodextrin molecules, such as aminocyclodextrin molecules, and the disaccharides of the cross-linked hyaluronic acid of the resulting hyaluronic acid product may have a molar ratio of 0.1-50%, preferably 1-20%, and more preferably 8-12%.

In the process, the activation of the hyaluronic acid and the coupling of the cyclodextrin molecule to the activated hyaluronic acid may occur simultaneously in step (b). Alternatively, the activation of the hyaluronic acid occurs prior to and separately from the coupling of the cyclodextrin molecule to the activated hyaluronic acid in step (b).

Preferred peptide coupling reagents are selected from the group consisting of triazine-based coupling reagents, carbodiimide coupling reagents, imidazolium-derived coupling reagents, Oxyma and COMU. Various other useful peptide coupling reagents are well known to the skilled person.

A particularly preferred group of peptide coupling reagent are triazine-based coupling reagents, such as 4-(4,6-dimethoxy-1,3,5-triazin-2-yl)-4-methylmorpholinium chloride (DMTMM) and 2-chloro-4,6-dimethoxy-1,3,5-triazine (CDMT). A particularly preferred triazine-based coupling reagent is DMTMM.

In a preferred embodiment, the amide bonds are formed between the amino group of amino-cyclodextrins having 6-8 glucopyranoside units and the carboxylic acid group of hyaluronic acid, followed by cross-linking with dextran and size reduction of the resulting gel to provide ether cross-linked dextran/hyaluronic acid gel particles with grafted cyclodextrins. This amide bond is realized by a peptide coupling agent, such as DMTMM.

The insights presented herein are also useful in a process of preparing a formulation of a guest molecule capable of forming a guest-host complex with a cyclodextrin host molecule, comprising the steps:
(a) providing (i) hyaluronic acid, (ii) dextran and (ii) one or more cyclodextrin host molecules capable of forming a guest-host complex with the guest molecule;
(b) grafting the one or more cyclodextrin host molecules onto the hyaluronic acid to form a cyclodextrin-grafted hyaluronic acid; and
c) cross-linking the cyclodextrin-grafted hyaluronic acid to the dextran using a bi- or polyfunctional cross-linking agent to form a cross-linked hydrogel.
d) bringing a solution of the guest molecule into contact with the cyclodextrin host molecules grafted onto the hyaluronic acid under conditions allowing for the formation of a guest-host complex between the cyclodextrin host molecules and the guest molecule, and optionally
e) recovering the guest-host complex bound to the cross-linked hydrogel.

Non-limiting examples of pharmaceutical agents and cyclodextrins capable of forming guest-host complexes are provided in tables 1-3.

| Table 1. Compiled from A. Magnúsdóttir, M. Másson and T. Loftsson, J. Incl. Phenom. Macrocycl. Chem. 44. 213-218, 2002 | |
|---|---|
| Cyclodextrin type | Drugs |
| α-Cyclodextrin | Alprostadil (PGE1) |
| | Cefotiam hexetil HCl |
| β-Cyclodextrin | Benexate HCl |
| | Dexamethasone |
| | Iodine |
| | Nicotine |
| | Nimesulide |
| | Nitroglycerin |
| | Omeprazol |
| | PGE2 |
| | Piroxicam |
| | Tiaprofenic acid |
| 2-Hydroxypropyl-β-cyclodextrin | Cisapride |
| | Hydrocortisone |
| | Indomethacin |
| | Itraconazole |
| | Mitomycin I |
| Randomly methylated β-cyclodextrin | 17β-Estradiol |
| | Chloramphenicol |
| Sulfobutylether β-cyclodextrin | Voriconazole |
| | Ziprasidone maleate |
| 2-Hydroxypropyl-γ-cyclodextrin | Diclofenac sodium |

| Table 2. Compiled from Amber Vyas, Shailendra Saraf, Swarnlata Saraf J. Incl. Phenom. Macrocvcl. Chem. (2008) 62:23-42 | |
|---|---|
| Cyclodextrin type | Drugs |
| β-CD, HP-β-CD | Ketoprofen |
| HP-β-CD, DM-β-CD, OM-β-CD | Gonadorelin, Leuprolide acetate, Recombinant human growth hormone, Lysozyme |
| β-CD, HP-β-CD | Niclosamide |
| β-CD | polypropylene glycol) bisamine |
| β-CD | Dexamethasone, Flurbiprofen, Doxorubicin hydrochloride |
| 2-HP-β-CD | Glutathione |
| HP-α-CD, HP-β-CD | Triclosan, Furosemide |
| α-CD, β-CD, γ-CD | Insulin |
| β-CD, M-β-CD, HP-β-CD, SB-β-CD | Estradiol |
| γ-CDC6 | Progesterone |
| HP-β-CD | Nifedipine |
| HP-β-CD | Hydrocortisone |
| 2-HP-β-CD | Insulin |
| HP-β-CD | Carvedilol |
| HP-β-CD | Insulin |
| β-CD hydrate | Amlodipine |
| HP-β-CD | Methoxydibenzoylmethane |
| HP-β-CD | Insulin |
| β-CDMCT | Octyl methoxycinnamate |
| Heptakis-β-CD | TPPS |
| HP-β-CD | Saquinavir |
| β-CD, 2-HP-β-CD | Hydrocortisone, Progesterone |
| Bis-CD | Bovine serum albumin |
| HP-β-CD | Bovine serum albumin |
| a, b, γ-CD | Gabexate Mesylate |
| β-CDC6 | Tamoxifen citrate |
| HP-β-CD | Itraconazole |
| a, b, γ-CD | Indomethacin, Furosemide, Naproxen |
| β-CD, HP-β-CD | Nifedipine |
| β-CD | Amikacin |
| HP-β-CD, γ-CD, RM-β-CD | Methacholine |
| (SBE)7m-β-CD | Chlorpromazine hydrochloride |
| α-Cyclodextrin | Isotretinoin |
| MCT-β-CD | Miconazole |
| SBE7-β-CD | Carbamazepine |
| β-CD | Retinoic acid |
| HP-β-CD | Rh-interferon α-2a |
| α-cyclodextrin | Droepiandrosterone |
| β-CD, HP-β-CD, Me-β-CD | Flurbiprofen |
| β-CD | Naproxen, Ibuprofen |
| β-CD, Me-β-CD | Piroxicam |
| α-CD, β-CD, HP-β-CD, RAME-β-CD | Melarsoprol |
| HP-β-CD, PM-β-CD | Bupranolol |
| β-CD | Diclofenac |

| Table 3. Compiled from R. Arun et al. Sci Pharm. 2008: 76: 567-598. | |
|---|---|
| Cyclodextrin type | Drugs |
| β-CD | Nimesulide, Sulfomethiazole, Lorazepam, Ketoprofen, Griseofulvin, Praziquantel, Chlorthalidon, Exodolac, Piroxicam, Itraconazole, Ibuprofen |
| α-CD | Praziquantel |
| γ-CD | Praziquantel, Omeprazole, Digoxin |
| HP-β-CD | Albendazole, DY-9760e, ETH-615, Levemopamil HCI, Sulfomethiazole, Ketoprofen, Griseofulvin, Itraconazole, Carbamazepine Zolpidem, Phenytoin, Rutin |
| DM-β-CD | Naproxen, Camptothesin |
| SBE-β-CD | DY-9760e, Danazol, Fluasterone, Spiranolactone |
| RM-β-CD | ETH-615, Tacrolimus |
| Randomly acetylated amorphous-β-CD | Naproxen |
| HP-β-CD, DM-β-CD | Promethazine |
| HP-β-CD | 2-ethylhexyl p-(dimethylamino)benzoate |
| β-CD | Glibenclamide |
| β-CD | Diclofenac sodium |
| β-CD, HP-β-CD | Quinaril |
| HP-β-CD, HP-γ-CD | Doxorubicin |
| HP-β-CD | Acyl ester prodrugs of Ganciclovir |
| γ-CD | Digoxin |
| HP-β-CD | Rutin |
| RDM-β-CD | Camptothesin |
| SBE-β-CD, HP-β-CD | Melphalan and Carmustine |
| γ-CD, HP-γ-CD, HP-β-CD | Paclitaxel |
| SBE-α-CD, SBE-β-CD, HP-β-CD, γ-CD, β-CD | Spiranolactone |
| β-CD | Flutamide |
| β-CD | Ketoprofen, Griseofulvin, Terfenadine |
| HP-β-CD | Albendazole, Ketoprofen, Phenytoin, Gliclazide |
| SBE7-β-CD | Spiranolactone |
| DM-β-CD | Tacrolimus |
| M-β-CD | Albendazole |
| ME-β-CD | Phenytoin |
| β-CD | Terfanidine, Tolbutamide |
| HP-β-CD | Tolbutamide, Amylobarbitone |
| HP-β-CD | Flutamide |
| γ-CD | Digoxin |
| HP-β-CD | Rutin |
| HP-β-CD | Clomipramine, Testosterone |
| SBE7-β-CD, HP-β-CD | Danazole |
| β-CD | Piroxicam |
| DM-β-CD | Carbamazepine |
| γ-CD | Digoxin |
| β-CD, SBE-β-CD | Glibenclamide |
| HP-β-CD | Miconazole |
| E-β-CD, Glu-β-CD, Mal-β-CD, SBE-β-CD, HP-β-CD | Phenytoin |
| β-CD, γ-CD, DM-β-CD, SBE-β-CD, HP-β-CD | Spironolactone |
| β-CD, HP-β-CD | Tolbutamide |
| DM-β-CD | α-Tocopheryl; nicotinate |
| β-CD | Acyclovir |
| DM-β-CD, HP-β-CD | Diphenhydramine HCl |
| DM-β-CD | Cyclosporin A |

### Explanation of abbreviations in Table 1-3:

β-CD, Beta cyclodextrin; HP-β-CD, Hydroxypropyl beta cyclodextrin; DM-β-CD, 2,6-di-O-methyl beta cyclodextrin; OM-β-CD, 6-O-maltosyl beta cyclodextrin; 2HP-β-CD, 2-hydroxypropyl beta cyclodextrin; HP-α-CD, Hydroxypropyl alpha cyclodextrin; α-CD, Alpha cyclodextrin; γ-CD, Gamma cyclodextrin; M-β-CD, Methyl-β-cyclodextrin; SB-β-CD, Sulfobutyl beta cyclodextrin; γ-CDC6, Gamma cyclodextrin C6 or amphiphilic 2,3-di-O-hexanoyl gamma cyclodextrin; β-CDMCT, Monochlorotriazinyl beta cyclodextrin; Heptakis-β-CD, Heptakis (2-x-amino-O-oligo (ethylene oxide)-6-hexylthio) beta cyclodextrin; bis-CDs, Ethylenediamino or diethylenetriamino bridged bis(beta cyclodextrin)s; RMβ-CD, randomly methylated beta cyclodextrin; (SBE)7m-β-CD, Sulfobutyl ether-β-cyclodextrin; MCT-β-CD, Monochlorotriaziny beta cyclodextrin; Me-β-CD, Methyl beta cyclodextrin; SBE-β-CD, Sulfobutylether-β-cyclodextrin; TPPS, Anionic 5,10,15,20-tetrakis(4-sulfonatophenyl)-21H,23H-porphyrin; E-β-CD, β-Cyclodextrin epichlorohydrin polymer; Glu-β-CD, Glucosyl-β-cyclodextrin; Mal-β-CD, Maltosyl-β-cyclodextrin.

Without desiring to be limited thereto, the present invention will in the following be illustrated by way of examples.

### Examples

### Example 1 - Grafting of 6-amino-gamma-CD to HA. followed by cross-linking to dextran

DMTMM (0.3 g) and 6-amino-gamma-CD (0.8 g) are dissolved in phosphate-buffered saline (PBS) (25 mL), and the pH of the solution is adjusted to approx. 6.5 (1.2 M HCI). This solution is added to dry HA powder (0.3 g) and then stirred gently. The reaction is heated to 45°C for 24 h and then allowed to cool down to room temperature. The product is washed twice with PBS (15 mL/g gel) and filtrated. The product is then washed three times with ethanol 70 % (15 mL/g gel) and the solution is discarded.

Dextran powder (0.1 g) is dissolved in PBS (25 ml), and the pH of the solution is adjusted to approx. 9.0 using NaOH. This solution is added to the cyclodextrin-grafted HA and then stirred gently. Cross-linking reagent BDDE is added and allowed to react with the the polymer mixture in solution, forming a gel. The gel is washed twice with PBS (15 mL/g gel) and filtrated. The gel is then washed three times with ethanol 70 % (15 mL/g gel) and the solution is discarded. Finally, pure ethanol is added to the gel and filtrated and dried under vacuum to yield the final material.

### Example 2- Characterization of HA-cyclodextrin-dextran product

The HA-cyclodextrin-dextran gel obtained in Example 1 is characterized by the swelling, i.e. the ability to absorb water, and the viscoelastic properties. Swelling is expressed as the amount of water in gram that one gram dry cross-linked HA-dextran polymer can absorb. The viscoelastic properties are measured by rheometry, and are expressed as the storage modulus (G') and the loss modulus (G").

The chemical composition of the HA-cyclodextrin-dextran is obtained by proton NMR spectroscopy after degradation of the HA polysaccharide strands by hylauronidase or equivalent to obtain sharp lines in the spectrum enabling proper quantification.

The chemical linking between HA, dextran and cyclodextrin is characterized by size exclusion chromatography coupled to mass spectrometry after degradation by both hylauronidase and dextranase or equivalent.

### Example 3 - Grafting of 3-amino-gamma-CD to HA, followed by cross-linking to dextran

### Step 1 : Grafting of cyclodextrin to hyaluronan

The 3-amino-γ-cyclodextrin was dissolved together with the activation agent DMTMM in 1 mM PBS buffer. 1 MDa Hyaluronan (HA) was dissolved in the same solution. The reaction mixture was stirred and allowed to react. The reaction was stopped by the addition of ethanol under vigorous stirring. The precipitate was isolated by decantation, washed with 70% ethanol (w/w) then with pure ethanol and dried in a vacuum oven at 23 °C over night.

The cyclodextrin conjugated hyaluronan was analysed by ¹H-NMR after enzymatic degradation of the polymer with chondroitinase ABC. A modification degree (cyclodextrin per hyaluronan disaccharide, (MOD CDx/HAdi)) of 8 to 12 % was obtained.

### Step 2: Formation of a hydrogel by cross-linking a mixture of modified hyaluronan and dextran.

Cyclodextrin-conjugated HA having a modification degree (MOD CDx/HAdi) of 12 % was placed together with Dextran 250 kDa in a flask and mixed with a solution of BDDE dissolved in 1 % (w/w) NaOH. The solution was stirred until a gel was obtained, and the resulting gel was passed through a grid for particle size reduction. The resulting particles were allowed to swell in a 10 mM PBS solution.

### Results

¹H-NMR after enzymatic degradation of the gel showed that the cyclodextrin content remained constant. Signals from cyclodextrin and dextran overlap in the ¹H-NMR spectrum.

| Table 4. Modification data based on integrated ¹H-NMR signals. | | |
|---|---|---|
| | MOD CDx/HAdi* [% mol/mol] | Dextran/Hyaluronan** [% mol/mol] |
| Gel prototype 1 | 12 | 10 |
| * The cyclodextrin is related to the hyaluronan disaccharide. | | |
| ** The dextran disaccharide is related to the hyaluronan disaccharide. | | |

## Claims

1. A hydrogel product comprising (a) one or more cyclodextrin molecules grafted to hyaluronic acid and (b) dextran, wherein the cyclodextrin-grafted hyaluronic acid is cross-linked to the dextran.

2. A hydrogel product according to claim 1, wherein the cyclodextrin-grafted hyaluronic acid is cross-linked to the dextran by ether bonds or by amide bonds.

3. A hydrogel product according to any one of claims 1-2, wherein the dextran and the hyaluronic acid chains are cross-linked to each other via a linking group which is derived from a bi- or polyfunctional cross-linking agent, such as a bis- or polyepoxide; preferably wherein said bi- or polyfunctional cross-linking agent is selected from the group consisting of 1,4-butanediol diglycidyl ether (BDDE), 1,2-bis(2,3-epoxypropoxy)ethylene (EGDGE) and ethylene glycol diglycidyl ether (EGDE), 1,2-ethanediol diglycidyl ether (EDDE) and diepoxyoctane; and more preferably wherein said bi- or polyfunctional cross-linking agent is 1,4-butanediol diglycidyl ether (BDDE).

4. A hydrogel product according to any one of claims 1-3, further comprising a guest molecule capable of forming a guest-host complex with the cyclodextrin molecule acting as a host; preferably wherein the guest molecule is selected from drugs and/or biologically active substances used in the treatment of disorders in the field of dermatology, aesthetics, ophthalmology, gynaecology, oncology, angiology, neurology, orthopaedics, rheumatology or aesthetic dermatology, such as anti-infective agents, antimicrobials, anti-inflammatory agents, cytostatic, cytotoxic, antiviral, anaesthetic, hemostatic, vasoconstrictor agents or growth factors; and more preferably wherein the guest molecule is a vitamin A molecule and more preferably retinol or retinoic acid, or an ester or an ether thereof.

5. A hydrogel product according to claim 4 for use as a medicament.

6. Use of the hydrogel product according to claim 4 for the preparation of pharmaceutical forms or medical devices involving the transport or the controlled release of said guest molecule.

7. A process of preparing a hydrogel product, comprising the steps of:
(a) providing (i) hyaluronic acid, (ii) dextran, and (iii) one or more cyclodextrin molecules;
(b) grafting the one or more cyclodextrin molecules onto the hyaluronic acid to form a cyclodextrin-grafted hyaluronic acid; and
(c) cross-linking the cyclodextrin-grafted hyaluronic acid to the dextran using a bi- or polyfunctional cross-linking agent.

8. A process according to claim 7, wherein the cross-linking of step (c) provides ether bonds between the dextran and the cyclodextrin-grafted hyaluronic acid.

9. A process according to any one of claims 7-8, wherein the grafting of step (b) provides amide bonds between the one or more cyclodextrin molecules and the hyaluronic acid.

10. A process according to claim 9, wherein the one or more cyclodextrin molecules of step (a) contain a linking group having an amino group, and wherein the linking group of the cyclodextrin molecule forms said amide bond with a carboxyl group of the hyaluronic acid, and, wherein the grafting of step (b) involves:
(i) activating the carboxyl groups on the hyaluronic acid with a peptide coupling reagent to form an activated hyaluronic acid; and
(ii) coupling the linking group of the one or more cyclodextrin molecules to the carboxyl groups of the activated hyaluronic acid by amide bonds; preferably wherein the peptide coupling reagent is selected from the group consisting of triazine-based coupling reagents, carbodiimide coupling reagents, imidazolium-derived coupling reagents, Oxyma and COMU; and more preferably wherein the peptide coupling reagent is a triazine-based coupling reagent.

11. A process according to claim 10, wherein the triazine-based coupling reagent is selected from the group consisting of 4-(4,6-dimethoxy-1,3,5-triazin-2-yl)-4-methylmorpholinium chloride (DMTMM) and 2-chloro-4,6-dimethoxy-1,3,5-triazine (CDMT); preferably wherein the triazine-based coupling reagent is DMTMM.

12. A process according to any one of claims 7-11, wherein the bi- or polyfunctional crosslinking agent of step (c) is a bis- or polyepoxide; preferably wherein said bi- or polyfunctional cross-linking agent is selected from the group consisting of 1,4-butanediol diglycidyl ether (BDDE), 1,2-bis(2,3-epoxypropoxy)ethylene (EGDGE) and ethylene glycol diglycidyl ether (EGDE), 1,2-ethanediol diglycidyl ether (EDDE) and diepoxyoctane; more preferably wherein said bi- or polyfunctional cross-linking agent is 1,4-butanediol diglycidyl ether (BDDE).

13. A process according to any one of claims 7-12, comprising the further steps:
d) bringing a solution of a guest molecule into contact with the cyclodextrin host molecules grafted onto the hyaluronic acid under conditions allowing for the formation of a guest-host complex between the cyclodextrin host molecules and the guest molecule, and optionally
e) recovering the guest-host complex bound to the cross-linked hydrogel.

14. A process according to claim 13, wherein the guest molecule is selected from drugs and/or biologically active substances used in the treatment of disorders in the field of dermatology, aesthetics, ophthalmology, gynaecology, oncology, angiology, neurology, orthopaedics, rheumatology or aesthetic dermatology, such as anti-infective agents, antimicrobials, anti-inflammatory agents, cytostatic, cytotoxic, antiviral, anaesthetic, hemostatic, vasoconstrictor agents or growth factors; preferably wherein the guest molecule is a vitamin A molecule, and more preferably retinol or retinoic acid, or an ester or an ether thereof.

## Patentansprüche

1. Hydrogelprodukt, umfassend (a) ein oder mehrere Cyclodextrinmoleküle, die auf Hyaluronsäure gepfropft sind, und (b) Dextran, wobei die Cyclodextrin-gepfropfte Hyaluronsäure mit dem Dextran vernetzt ist.

2. Hydrogelprodukt nach Anspruch 1, wobei die Cyclodextrin-gepfropfte Hyaluronsäure mit dem Dextran durch Etherbindungen oder durch Amidbindungen vernetzt ist.

3. Hydrogelprodukt nach einem der Ansprüche 1-2, wobei die Dextran- und die Hyaluronsäureketten miteinander über eine Verknüpfungsgruppe vernetzt sind, die von einem bi- oder polyfunktionellen Vernetzungsmittel, wie einem Bis- oder Polyepoxid, herrührt, wobei vorzugsweise das bi- oder polyfunktionelle Vernetzungsmittel aus der Gruppe ausgewählt ist, bestehend aus 1,4-Butandioldiglycidylether (BDDE), 1,2-Bis(2,3-epoxypropoxy)ethylen (EGDGE) und Ethylenglykoldiglycidylether (EGDE), 1,2-Ethandioldiglycidylether (EDDE) und Diepoxyoctan, und wobei stärker bevorzugt das bi- oder polyfunktionelle Vernetzungsmittel 1,4-Butandioldiglycidylether (BDDE) ist.

4. Hydrogelprodukt nach einem der Ansprüche 1-3, das ferner ein Gastmolekül umfasst, das einen Gast-Wirt-Komplex mit dem als Wirt dienenden Cyclodextrinmolekül bilden kann, wobei vorzugsweise das Gastmolekül aus Arzneistoffen und/oder biologischen Wirkstoffen ausgewählt ist, die bei der Behandlung von Störungen auf dem Gebiet der Dermatologie, Ästhetik, Ophthalmologie, Gynäkologie, Onkologie, Angiologie, Neurologie, Orthopädie, Rheumatologie oder ästhetischen Dermatologie verwendet werden, wie Antiinfektiva, antimikrobielle Mittel, entzündungshemmende Mittel, zytostatische, zytotoxische, antivirale, anästhetische, hämostatische, vasokonstriktorische Mittel oder Wachstumsfaktoren, und wobei stärker bevorzugt das Gastmolekül ein Vitamin A-Molekül und stärker bevorzugt Retinol oder Retinsäure oder ein Ester oder ein Ether davon ist.

5. Hydrogelprodukt nach Anspruch 4 zur Verwendung als Medikament.

6. Verwendung des Hydrogelprodukts nach Anspruch 4 zur Herstellung pharmazeutischer Formen oder medizinischer Vorrichtungen, die den Transport oder die kontrollierte Freisetzung des Gastmoleküls beinhalten.

7. Verfahren zur Herstellung eines Hydrogelprodukts, umfassend die folgenden Schritte:
(a) Bereitstellen von (i) Hyaluronsäure, (ii) Dextran und (iii) einem oder mehreren Cyclodextrinmolekülen;
(b) Pfropfen des einen oder der mehreren Cyclodextrinmoleküle auf die Hyaluronsäure, um eine Cyclodextrin-gepfropfte Hyaluronsäure herzustellen, und
(c) Vernetzen der Cyclodextrin-gepfropften Hyaluronsäure mit dem Dextran unter Verwendung eines bi- oder polyfunktionellen Vernetzungsmittels.

8. Verfahren nach Anspruch 7, wobei das Vernetzen von Schritt (c) Etherbindungen zwischen dem Dextran und der Cyclodextrin-gepfropften Hyaluronsäure liefert.

9. Verfahren nach einem der Ansprüche 7-8, wobei das Pfropfen von Schritt (b) Amidbindungen zwischen dem einen oder den mehreren Cyclodextrinmolekülen und der Hyaluronsäure liefert.

10. Verfahren nach Anspruch 9, wobei das eine oder die mehreren Cyclodextrinmoleküle von Schritt (a) eine Verknüpfungsgruppe mit einer Aminogruppe enthalten und wobei die Verknüpfungsgruppe des Cyclodextrinmoleküls die Amidbindung mit einer Carboxylgruppe der Hyaluronsäure bildet und wobei das Pfropfen von Schritt (b) Folgendes einschließt:
(i) Aktivieren der Carboxylgruppen an der Hyaluronsäure mit einem Peptidkupplungsreagenz, so dass eine aktivierte Hyaluronsäure hergestellt wird, und
(ii) Kuppeln der Verknüpfungsgruppe des einen oder der mehreren Cyclodextrinmoleküle an die Carboxylgruppen der aktivierten Hyaluronsäure durch Amidbindungen, wobei vorzugsweise das Peptidkupplungsreagenz aus der Gruppe ausgewählt ist, bestehend aus Kupplungsreagenzien auf Triazin-Basis, Carbodiimid-Kupplungsreagenzien, von Imidazolium stammenden Kupplungsreagenzien, Oxyma und COMU, und wobei stärker bevorzugt das Peptidkupplungsreagenz ein Kupplungsreagens auf Triazin-Basis ist.

11. Verfahren nach Anspruch 10, wobei das Kupplungsreagenz auf Triazin-Basis aus der Gruppe ausgewählt ist, bestehend aus 4-(4,6-Dimethoxy-1,3,5-triazin-2-yl)-4-methylmorpholiniumchlorid (DMTMM) und 2-Chlor-4,6-dimethoxy-1,3,5-triazin (CDMT), wobei vorzugsweise das Kupplungsreagenz auf Triazin-Basis DMTMM ist.

12. Verfahren nach einem der Ansprüche 7-11, wobei das bi- oder polyfunktionelle Vernetzungsmittel von Schritt (c) ein Bis- oder Polyepoxid ist, wobei vorzugsweise das bi- oder polyfunktionelle Vernetzungsmittel aus der Gruppe ausgewählt ist, bestehend aus 1,4-Butandioldiglycidylether (BDDE), 1,2-Bis(2,3-epoxypropoxy)-ethylen (EGDGE) und Ethylenglykoldiglycidylether (EGDE), 1,2-Ethandioldiglycidylether (EDDE) und Diepoxyoctan, wobei stärker bevorzugt das bi- oder polyfunktionelle Vernetzungsmittel 1,4-Butandioldiglycidylether (BDDE) ist.

13. Verfahren nach einem der Ansprüche 7-12, umfassend die folgenden weiteren Schritte:
d) Inkontaktbringen einer Lösung des Gastmoleküls mit den auf die Hyaluronsäure gepfropften Cyclodextrin-Wirtsmolekülen unter Bedingungen, die die Bildung eines Gast-Wirt-Komplexes zwischen den Cyclodextrin-Wirtsmolekülen und dem Gastmolekül gestatten, und gegebenenfalls
e) Gewinnen des an das vernetzte Hydrogel gebundenen Gast-Wirt-Komplexes.

14. Verfahren nach Anspruch 13, wobei das das Gastmolekül aus Arzneistoffen und/oder biologischen Wirkstoffen ausgewählt ist, die bei der Behandlung von Störungen auf dem Gebiet der Dermatologie, Ästhetik, Ophthalmologie, Gynäkologie, Onkologie, Angiologie, Neurologie, Orthopädie, Rheumatologie oder ästhetischen Dermatologie verwendet werden, wie Antiinfektiva, antimikrobielle Mittel, entzündungshemmende Mittel, zytostatische, zytotoxische, antivirale, anästhetische, hämostatische, vasokonstriktorische Mittel oder Wachstumsfaktoren, und wobei stärker bevorzugt das Gastmolekül ein Vitamin A-Molekül und stärker bevorzugt Retinol oder Retinsäure oder ein Ester oder ein Ether davon ist.

## Revendications

1. Produit d'hydrogel comprenant (a) une ou plusieurs molécules de cyclodextrine greffées à l'acide hyaluronique et (b) du dextrane, dans lequel l'acide hyaluronique greffé avec la cyclodextrine est réticulé avec le dextrane.

2. Produit d'hydrogel selon la revendication 1, dans lequel l'acide hyaluronique greffé avec la cyclodextrine est réticulé avec le dextrane par des liaisons éther ou par des liaisons amide.

3. Produit d'hydrogel selon l'une quelconque des revendications 1 à 2, dans lequel les chaînes de dextrane et d'acide hyaluronique sont réticulées les unes aux autres par l'intermédiaire d'un groupe de liaison qui est dérivé d'un agent de réticulation bi- ou polyfonctionnel, tel qu'un bis- ou polyépoxyde ; de préférence dans lequel ledit agent de réticulation bi- ou polyfonctionnel est choisi dans le groupe constitué des éther diglycidylique de 1,4-butanediol (BDDE), éther diglycidylique de 1,2-bis(2,3-époxypropoxy)éthylène (EGDGE) et d'éthylène glycol (EGDE), éther diglycidylique de 1,2-éthanediol (EDDE) et diépoxyoctane ; et, plus préférablement, dans lequel ledit agent de réticulation bi- ou polyfonctionnel est l'éther diglycidylique de 1,4-butanediol (BDDE).

4. Produit d'hydrogel selon l'une quelconque des revendications 1 à 3, comprenant en outre une molécule incluse capable de former un complexe inclus-hôte avec la molécule de cyclodextrine agissant en tant qu'hôte ; de préférence dans lequel la molécule incluse est choisie parmi des médicaments et/ou des substances biologiquement actives utilisées dans le traitement de troubles dans le domaine de la dermatologie, de l'esthétique, de l'ophtalmologie, de la gynécologie, de l'oncologie, de l'angiologie, de la neurologie, de l'orthopédie, de la rhumatologie ou de la dermatologie esthétique, tels que des agents anti-infectieux, des antimicrobiens, des agents anti-inflammatoires, des agents cytostatiques, cytotoxiques, antiviraux, anesthésiques, hémostatiques, vasoconstricteurs ou des facteurs de croissance ; et plus préférablement dans lequel la molécule incluse est une molécule de vitamine A et, plus préférablement, le rétinol ou l'acide rétinoïque, ou un ester ou un éther de ceux-ci.

5. Produit d'hydrogel selon la revendication 4 pour utilisation en tant que médicament.

6. Utilisation du produit d'hydrogel selon la revendication 4 pour la préparation de formes pharmaceutiques ou de dispositifs médicaux mettant en oeuvre le transport ou la libération contrôlée de ladite molécule incluse.

7. Procédé de préparation d'un produit d'hydrogel, comprenant les étapes de :
(a) fourniture de (i) acide hyaluronique, (ii) dextrane, et (iii) une ou plusieurs molécules de cyclodextrine ;
(b) greffage des une ou plusieurs molécules de cyclodextrine sur l'acide hyaluronique pour former un acide hyaluronique greffé avec la cyclodextrine ; et
(c) réticulation de l'acide hyaluronique greffé avec la cyclodextrine avec du dextrane au moyen d'un agent de réticulation bi- ou polyfonctionnel.

8. Procédé selon la revendication 7, dans lequel la réticulation de l'étape (c) produit des liaisons éther entre le dextrane et l'acide hyaluronique greffé avec la cyclodextrine.

9. Procédé selon l'une quelconque des revendications 7 à 8, dans lequel le greffage de l'étape (b) produit des liaisons amide entre les une ou plusieurs molécules de cyclodextrine et l'acide hyaluronique.

10. Procédé selon la revendication 9, dans lequel les une ou plusieurs molécules de cyclodextrine de l'étape (a) contiennent un groupe de liaison comportant un groupe amino, et dans lequel le groupe de liaison de la molécule de cyclodextrine forme ladite liaison amide avec un groupe carboxyle de l'acide hyaluronique, et, dans lequel le greffage de l'étape (b) met en oeuvre :
(i) l'activation des groupes carboxyle sur l'acide hyaluronique avec un réactif de couplage peptidique pour former un acide hyaluronique activé ; et
(ii) le couplage du groupe de liaison des une ou plusieurs molécules de cyclodextrine aux groupes carboxyle de l'acide hyaluronique activé par des liaisons amide ; de préférence dans lequel le réactif de couplage peptidique est choisi dans le groupe constitué de réactifs de couplage à base de triazine, réactifs de couplage de carbodiimide, réactifs de couplage dérivés d'imidazolium, Oxyma et COMU ; et plus préférablement dans lequel le réactif de couplage peptidique est un réactif de couplage à base de triazine.

11. Procédé selon la revendication 10, dans lequel le réactif de couplage à base de triazine est choisi dans le groupe constitué des chlorure de 4-(4,6-diméthoxy-1,3,5-triazin-2-yl)-4-méthylmorpholinium (DMTMM) et 2-chloro-4,6-diméthoxy-1,3,5-triazine (CDMT) ; de préférence dans lequel le réactif de couplage à base de triazine est DMTMM.

12. Procédé selon l'une quelconque des revendications 7 à 11, dans lequel l'agent de réticulation bi- ou polyfonctionnel de l'étape (c) est un bis- ou polyépoxyde ; de préférence dans lequel ledit agent de réticulation bi- ou polyfonctionnel est choisi dans le groupe constitué des éther diglycidylique de 1,4-butanediol (BDDE), éther diglycidylique de 1,2-bis(2,3-époxypropoxy)éthylène (EGDGE) et d'éthylène glycol (EGDE), éther diglycidylique de 1,2-éthanediol (EDDE) et diépoxyoctane ; plus préférablement dans lequel ledit agent de réticulation bi- ou polyfonctionnel est l'éther diglycidylique de 1,4-butanediol (BDDE).

13. Procédé selon l'une quelconque des revendications 7 à 12, comprenant les étapes supplémentaires de :
d) mise en contact d'une solution d'une molécule incluse avec les molécules hôtes de cyclodextrine greffées sur l'acide hyaluronique dans des conditions permettant la formation d'un complexe inclus-hôte entre les molécules hôtes de cyclodextrine et la molécule incluse, et facultativement
e) récupération du complexe inclus-hôte lié à l'hydrogel réticulé.

14. Procédé selon la revendication 13, dans lequel la molécule incluse est choisie parmi des médicaments et/ou des substances biologiquement actives utilisées dans le traitement de troubles dans le domaine de la dermatologie, de l'esthétique, de l'ophtalmologie, de la gynécologie, de l'oncologie, de l'angiologie, de la neurologie, de l'orthopédie, de la rhumatologie ou de la dermatologie esthétique, tels que des agents anti-infectieux, des antimicrobiens, des agents anti-inflammatoires, des agents cytostatiques, cytotoxiques, antiviraux, anesthésiques, hémostatiques, vasoconstricteurs ou des facteurs de croissance ; et plus préférablement dans lequel la molécule incluse est une molécule de vitamine A et, plus préférablement, le rétinol ou l'acide rétinoïque, ou un ester ou un éther de ceux-ci.
